# EUROPÄISCHE PATENTSCHRIFT

(11) **EP 3 459 478 B1**
(45) Veröffentlichungstag und Bekanntmachung des Hinweises auf die Patenterteilung: **29.07.2020**
(21) Anmeldenummer: 18195824.0
(22) Anmeldetag: 20.09.2018
(51) Int. Cl.: A61B 17/88

(54) **MEDIZINTECHNISCHE VERFORMUNGSEINRICHTUNG, VERFORMUNGSSYSTEM UND VERFAHREN ZUM VERFORMEN EINES ARTIKELS**
MEDICAL DEFORMATION DEVICE, DEFORMATION SYSTEM AND METHOD FOR DEFORMING AN ARTICLE
DISPOSITIF DE MISE EN FORME TECHNIQUE MÉDICAL, SYSTÈME DE MISE EN FORME ET PROCÉDÉ DE MISE EN FORME D'UN ARTICLE

(30) Priorität: 25.09.2017 DE 102017122143
(43) Veröffentlichungstag der Anmeldung: 27.03.2019
(73) Patentinhaber: Aesculap AG, 78532 Tuttlingen (DE)
(72) Erfinder: Kozak, Josef, 78532 Tuttlingen (DE); Beger, Jens, 78532 Tuttlingen (DE); Weißhaupt, Dieter, 78194 Immendingen (DE)
(74) Vertreter: Hoeger, Stellrecht & Partner Patentanwälte mbB

(56) Entgegenhaltungen:
- WO-A1-2014/088801
- WO-A1-2017/037113
- DE-A1-102015 102 776
- US-A1- 2016 175 013
- US-B2- 7 957 831

## Beschreibung

Die Erfindung betrifft eine medizintechnische Verformungseinrichtung für einen medizintechnischen Artikel.

Außerdem betrifft die Erfindung ein medizintechnisches Verformungssystem und ein Verfahren zum Verformen eines medizintechnischen Artikels.

Es sind medizintechnische Fixationssysteme bekannt zum Fixieren von Körperstrukturen relativ zueinander, wobei an den Körperstrukturen festgelegte Verankerungselemente mittels eines Stabilisierungselementes miteinander verbunden werden. Ein beispielhaftes Anwendungsgebiet sind Stabilisierungen von Wirbelkörpern relativ zueinander, wobei als Verankerungselemente Knochenschrauben und als Stabilisierungselement ein Stab eingesetzt werden. Derartige Fixationssysteme sind zum Beispiel in der EP 2 910 206 A1 und der WO 2016/134911 A1 beschrieben. Bekannt ist es dabei, die Lage der Verankerungselemente, die an den Strukturen fixiert sind - d. h. beispielsweise Knochenschrauben, welche an den Wirbelkörpern festgelegt sind - relativ zueinander mittels eines medizintechnischen Navigationssystems zu ermitteln. Mit der Maßgabe, dass die Verankerungselemente in definierter Relativposition zueinander zu fixieren sind, kann vom Navigationssystem die Form des Stabilisierungselementes bestimmt werden. Hierbei kann zum Beispiel eine Raumkurve ermittelt werden. Anhand dieser Information kann ein Stabilisierungselement aus einem Vorrat ausgewählt werden. Alternativ oder ergänzend besteht die Möglichkeit, das Stabilisierungselement in die Form der Raumkurve zu überführen. Zu diesem Zweck ist der Einsatz von medizintechnischen Verformungseinrichtungen beispielsweise aus der WO 2016/134911 A1 bekannt. Die darin beschriebene Verformungseinrichtung bewährt sich in der Praxis.

WO 2017/037113 A1 wird als nächstliegender Stand der Technik gegenüber dem Gegenstand des Anspruchs 1 angesehen. Es offenbart ein medizintechnische Verformungseinrichtung für einen medizintechnischen Artikel, die eine Aufnahme zum Fixieren des Artikels aufweist und ein Verformungswerkzeug, mit dem zum Verformen auf den Artikel eingewirkt wird, wobei das Verformungswerkzeug in definierter Weise relativ zur Aufnahme bewegbar ist und am Verformungswerkzeug eine von einem Navigationssystem erfassbare medizintechnische Markiereinrichtung gehalten ist zum Bestimmen einer Relativposition des Verformungswerkzeugs und der Aufnahme.

Die vorliegende Erfindung bezieht sich auf eine medizintechnische Verformungseinrichtung für einen medizintechnischen Artikel, der nicht auf ein Stabilisierungselement und insbesondere einen Stab eines medizintechnischen Fixationssystems beschränkt ist.

Aufgabe der vorliegenden Erfindung ist es, eine Verformungseinrichtung, ein Verformungssystem und ein Verfahren der eingangs genannten Art bereitzustellen, mit der bzw. dem die Verformung eines medizintechnischen Artikels erleichtert ist.

Diese Aufgabe wird durch eine erfindungsgemäße medizintechnische Verformungseinrichtung für einen medizintechnischen Artikel gelöst, die eine Aufnahme zum Fixieren des Artikels aufweist und ein Verformungswerkzeug, mit dem zum Verformen auf den Artikel eingewirkt wird, wobei das Verformungswerkzeug in definierter Weise relativ zur Aufnahme bewegbar ist und an der Aufnahme und am Verformungswerkzeug jeweils eine von einem Navigationssystem erfassbare medizintechnische Markiereinrichtung gehalten ist zum Bestimmen einer Relativposition des Verformungswerkzeugs und der Aufnahme, und wobei am Verformungswerkzeug eine weitere Markiereinrichtung festgelegt ist zum Bestimmen, mittels des Navigationssystems, eines Ausmaßes einer Verformung des Artikels.

Der vorliegenden Erfindung liegt die Überlegung zugrunde, dass, um dem medizintechnischen Artikel eine Sollform zu verleihen, die Position und das Ausmaß der Verformung unter Einsatz eines medizintechnischen Navigationssystems auf verhältnismäßig einfache Weise überprüft werden können. Der Artikel kann an der Aufnahme festgelegt werden, und zur Verformung kommt das Verformungswerkzeug zum Einsatz. Die Relativposition der Aufnahme und des Verformungswerkzeuges kann anhand eines Navigationssystems beispielsweise dadurch ermittelt werden, dass die Lage und/oder Orientierung der an diesen gehaltenen Markiereinrichtungen vom Navigationssystem bestimmt werden können, woraus die Positionen der Aufnahme und des Verformungswerkzeuges abgeleitet werden können. Zu diesem Zweck sind die Markiereinrichtungen vorliegend unterschiedlich und voneinander unterscheidbar ausgestaltet. Dies gibt die Möglichkeit, das Verformungswerkzeug an einer Soll-Verformungsposition an dem Artikel zu positionieren, an der die Verformung vorgenommen wird. Zusätzlich ist eine weitere, von den vorliegend genannten Markiereinrichtungen unterscheidbare Markiereinrichtung vorhanden, um das Ausmaß der Verformung des Artikels festzustellen. Die weitere Markiereinrichtung ist am Verformungswerkzeug gehalten und ermöglicht es dem Navigationssystem festzustellen, ob die Verformung an der Soll-Verformungsposition mit einer Soll-Verformung übereinstimmt.

Die erfindungsgemäße Verformungseinrichtung kann insbesondere rein mechanisch ausgestaltet und auf diese Weise einfach handhabbar sein.

Vorteilhafterweise besteht die Möglichkeit, die Verformung des Artikels ohne a-priori-Kenntnis über die Soll-Verformungsposition und Soll-Verformung vorzunehmen. Bei Einnahme einer korrekten Soll-Verformungsposition und Soll-Verformung kann mittels des Navigationssystems, hierauf wird nachfolgend noch eingegangen, an einen Benutzer ein entsprechender Hinweis ausgegeben werden, so dass der Benutzer auf einfache Weise die Verformung ohne beispielsweise Eingabe von Verformungsdaten in die Verformungseinrichtung führen kann.

Das Verformungswerkzeug kann relativ zur Aufnahme beispielsweise verschieblich ausgestaltet sein. Hierbei kann eine insbesondere geradlinige Verschiebung vorgesehen sein, beispielsweise entlang oder parallel zu einer von der Aufnahme definierten Achse.

Günstig ist es, wenn die Verformungseinrichtung eine Halteeinrichtung umfasst, die die Aufnahme umfasst oder ausbildet oder an der die Aufnahme festgelegt ist, und dass das Verformungswerkzeug an der Halteeinrichtung beweglich gehalten ist, wobei bevorzugt eine feststellbar bewegliche Halterung des Verformungswerkzeugs an der Halteeinrichtung vorgesehen sein kann. Die Aufnahme ist an der Halteeinrichtung angeordnet, und das Verformungswerkzeug kann an der Halteeinrichtung gehalten relativ zur Aufnahme bewegt werden. Einer Bedienperson wird auf diese Weise die Handhabung der Verformungseinrichtung erleichtert.

Vorteilhafterweise umfasst oder bildet die Halteeinrichtung eine Führung für einen Lagerkörper des Verformungswerkzeuges. Durch die Führung wird es dem Benutzer erleichtert, das Verformungswerkzeug und die Aufnahme in eine definierte Relativposition zu bringen. Eine Markiereinrichtung der Verformungseinrichtung ist vorteilhafterweise am Lagerkörper gehalten.

Von Vorteil ist es, wenn die Verformungseinrichtung eine an der Halteeinrichtung gehaltene medizinische Markiereinrichtung umfasst zum Bestimmen der Lage und/oder Orientierung der Verformungseinrichtung relativ zum Navigationssystem. Die Markiereinrichtung, die sich insbesondere von den vorstehend genannten Markiereinrichtungen unterscheidet, ermöglicht es dem Navigationssystem, die Verformungseinrichtung in Lage und/oder Orientierung zu erfassen und insbesondere zu verfolgen. Dies erleichtert es dem Navigationssystem, anhand der weiteren Markiereinrichtungen die Soll-Verformungsposition und die Soll-Verformung zu überprüfen.

Es ist vorteilhafterweise vorgesehen, dass die Aufnahme relativ zur Halteeinrichtung drehbar ausgestaltet ist, wobei die Aufnahme vorzugsweise an der Halteeinrichtung drehbar gelagert ist. Günstig ist es dabei, wenn die Aufnahme eine Achse definiert, die mit einer vom in der Aufnahme fixierten längserstreckten Artikel definierten Achse fluchtet. Mittels der drehbaren Aufnahme relativ zur Halteeinrichtung wird die Verformung des Artikels erleichtert, denn es bleibt einem Benutzer erspart, erforderlichenfalls den Artikel aus der Aufnahme zu entfernen und in andersartiger Orientierung oder Position wieder in der Aufnahme zu fixieren. Stattdessen kann, insbesondere bei längserstrecktem und stabförmigem Artikel, lediglich die Aufnahme zu drehen sein. Der über die Drehung bereitgestellte Freiheitsgrad kann mittels der in der Aufnahme gehaltenen Markiereinrichtung vom Navigationssystem überwacht werden. Dies bietet nicht nur die Möglichkeit, die Soll-Verformungsposition in Bezug auf den Relativabstand des Verformungswerkzeuges und der Aufnahme zu kontrollieren, sondern auch in Bezug auf die Relativorientierung des Artikels und des Verformungswerkzeuges abhängig von einer Drehung.

Denkbar ist eine handbetätigte oder mittels eines Antriebs betätigte Drehung der Aufnahme relativ zur Halteeinrichtung. Die Verformungseinrichtung kann einen entsprechenden Antrieb umfassen.

In entsprechender Weise ist eine handbetätigte oder mittels eines Antriebs betätigte Bewegung und insbesondere Verschiebung des Verformungswerkzeugs relativ zur Aufnahme denkbar. Die Verformungseinrichtung kann einen entsprechenden Antrieb umfassen.

Bei einer vorteilhaften Ausführungsform kann vorgesehen sein, dass das Verformungswerkzeug ein Biegewerkzeug ist und einen Biegekörper und ein mit diesem gekoppeltes Betätigungselement umfasst, wobei der Artikel mittels des Biegekörpers gebogen werden kann. Diese Umsetzung ist beispielsweise zum Biegen eines Artikels in Gestalt eines Stabilisierungselementes und insbesondere Stabes von Vorteil. Mittels des Betätigungselementes kann auf den mit diesem gekoppelten Biegekörper und über diesen auf den Artikel eingewirkt werden.

Beispielsweise ist das Betätigungselement an einem Lagerkörper des Biegewerkzeugs drehbar und/oder verschieblich gelagert. Der Lagerkörper ist vorteilhafterweise verschieblich an einer Führung der vorstehend genannten Halteeinrichtung gelagert.

Günstig ist es, wenn das Verformungswerkzeug ein Anlageelement und insbesondere ein Auflageelement für den Artikel umfasst oder ausbildet. Der in der Aufnahme fixierte Artikel kann am Anlageelement angelegt und dadurch in verhältnismäßig stabiler Position am Verformungswerkzeug angeordnet werden, um dem Benutzer die Verformung und insbesondere Biegung des Artikels zu erleichtern.

Als vorteilhaft erweist es sich, wenn das Verformungswerkzeug ein verstellbares Anschlagelement für den Artikel umfasst oder ausbildet. Mittels des Anschlagelementes kann das Ausmaß der Verformung, beispielsweise über den vorstehend genannten Biegekörper, eingestellt und dadurch begrenzt werden. Das Anschlagelement kann durch das vorstehend genannte Anlageelement ausgebildet sein oder integral mit diesem gebildet sein. Vorteilhafterweise ist das Anschlagelement an einem Lagerkörper des Verformungswerkzeuges feststellbar beweglich gehalten. Der Benutzer kann durch Einstellung der Position des Anschlagelementes vorgeben, in welchem Ausmaß der Artikel mittels des Verformungswerkzeuges verformt wird.

Günstig ist es, wenn am Betätigungselement eine Markiereinrichtung des Verformungswerkzeuges gehalten ist. Mittels des Navigationssystems kann die Bewegung des Betätigungselementes und damit des Biegekörpers bestimmt werden. Hieraus kann die Verformung des Artikels ermittelt werden.

Alternativ oder ergänzend kann vorgesehen sein, dass am Anschlagelement eine Markiereinrichtung des Verformungswerkzeugs gehalten ist. Die Position des Anschlagelementes an der Verformungseinrichtung (beispielsweise relativ zum Lagerkörper, der Aufnahme und/oder Halteeinrichtung insgesamt) kann vom Navigationssystem bestimmt und auf diese Weise das zu erwartende Ausmaß der Verformung bestimmt werden.

Das Verformungswerkzeug kann ein von Hand oder mittels eines Antriebs betätigbares Verformungswerkzeug sein. Die Verformungseinrichtung kann einen entsprechenden Antrieb umfassen.

Die eingangs genannte Aufgabe wird durch ein medizintechnisches Verformungssystem gelöst, umfassend eine Verformungseinrichtung der vorstehend genannten Art und ein medizintechnisches Navigationssystem zum Erfassen und Verfolgen der Markiereinrichtungen der Verformungseinrichtung, wobei mit dem Navigationssystem die Relativposition der Aufnahme und des Verformungswerkzeugs ermittelbar ist zum Feststellen einer Verformungsposition des Artikels, und wobei mit dem Navigationssystem das Ausmaß der Verformung des Artikels an der Verformungsposition ermittelbar ist.

Das Navigationssystem kann anhand der Markiereinrichtungen wie vorstehend erläutert feststellen, welche Verformungsposition das Verformungswerkzeug einnimmt, insbesondere ob dieses an einer Soll-Verformungsposition angeordnet ist. Außerdem kann das Ausmaß der Verformung überprüft werden, ob dieses mit einer Soll-Verformung übereinstimmt. Zu diesem Zweck kann wie vorstehend erläutert insbesondere am Betätigungselement und/oder am Anschlagelement eine Markiereinrichtung gehalten sein.

Günstig ist es, wenn in einer Speichereinheit eine Sollform des Artikels gespeichert ist und wenn das Navigationssystem eine Hinweiseinrichtung umfasst oder ausbildet. An der Hinweiseinrichtung kann insbesondere mindestens ein Hinweis darüber ausgegeben werden, dass das Verformungswerkzeug eine Soll-Verformungsposition zur Aufnahme einnimmt, insbesondere beim Bewegen des Verformungswerkzeuges relativ zur Aufnahme. Ohne a-priori-Kenntnis kann der Benutzer beispielsweise beim Bewegen und insbesondere Verschieben des Verformungswerkzeuges darüber informiert werden, dass dieses die korrekte Position einnimmt. Ein Hinweis, zum Beispiel optisch, akustisch und/oder haptisch, kann an den Benutzer ausgegeben werden. Alternativ oder ergänzend kann der Hinweis darüber bereitgestellt werden, dass die mittels des Verformungswerkzeuges erzeugte oder erzeugbare Verformung des Artikels mit einer Soll-Verformung übereinstimmt. Beispielsweise wird die Bewegung des Bestätigungselementes vom Navigationssystem festgestellt und die sich infolgedessen ergebende Verformung bestimmt. Alternativ oder ergänzend kann die Position und/oder Bewegung des Anschlagelementes festgestellt und die zu erwartende Verformung bestimmt werden. In beiden Fällen kann der Benutzer über einen entsprechenden Hinweis informiert werden, dass die gewünschte Soll-Verformung erzielt ist oder erzielt werden wird.

Der Artikel kann an mehreren Positionen oder mehrfach verformt werden. Hierzu ist es günstig, wenn die Sollform vom Navigationssystem in einzelne Segmente unterteilbar ist, wobei an der Hinweiseinrichtung sequentiell Hinweise bereitstellbar sind, dass ein jeweiliges Segment durch Verformen des Artikels an einer Soll-Verformungsposition mit einer Soll-Verformung mit einer Sollform des jeweiligen Segmentes übereinstimmt. Der Artikel kann segmentweise verformt werden. Die korrekte Verformung jedes Segmentes kann vom Navigationssystem sequentiell überwacht, verifiziert und bestätigt werden.

Beispielsweise werden die Segmente sukzessive von distal nach proximal verformt, bezogen auf die Aufnahme.

Bei einer vorteilhaften Ausführungsform kann vorgesehen sein, dass vom Navigationssystem Verformungsinformationen an mindestens einen Antrieb der Verformungseinrichtung zum maschinellen Verformen des Artikels entsprechend der Verformungsinformationen übermittelbar sind. Es ist denkbar, dass eine Verformung des Artikels maschinell, ohne Zutun des Benutzers, durchgeführt werden kann.

Ein erfindungsgemäßes, die eingangs genannte Aufgabe lösendes Verfahren zum Verformen eines medizintechnischen Artikels sieht den Einsatz eines Verformungssystems der vorstehend genannten Art vor, wobei mit dem Navigationssystem eine Verformungsposition des zu verformenden medizintechnischen Artikels festgestellt wird und ein Ausmaß der Verformung des Artikels an der Verformungsposition ermittelt wird.

Die Vorteile des erfindungsgemäßen Verfahrens wurden bereits im Zusammenhang mit der Erläuterung der Verformungseinrichtung und des Verformungssystems erwähnt. Auf die vorangegangenen Ausführungen wird verwiesen. Vorteilhafte Ausführungsbeispiele des Verfahrens ergeben sich durch vorteilhafte Ausführungsformen der Verformungseinrichtung und des Verformungssystems.

Die nachfolgende Beschreibung bevorzugter Ausführungsformen der Erfindung dient im Zusammenhang mit der Zeichnung der näheren Erläuterung der Erfindung. Das nachfolgend beschriebene Verformungssystem erlaubt die Durchführung eines bevorzugten Ausführungsbeispiels eines erfindungsgemäßen Verfahrens. Es zeigen:
- Figur 1:: eine perspektivische schematische Darstellung eines erfindungsgemäßen Verformungssystems, umfassend ein Navigationssystem und eine bevorzugte Ausführungsform einer erfindungsgemäßen Verformungseinrichtung;
- Figur 2:: eine vergrößerte Darstellung der Verformungseinrichtung in Figur 1; und
- Figur 3:: eine weitere bevorzugte Ausführungsform der erfindungsgemäßen Verformungseinrichtung, die beispielsweise anstelle der oder zusätzlich zu der Verformungseinrichtung in Figur 1 Bestandteil des erfindungsgemäßen Verformungssystems sein kann.

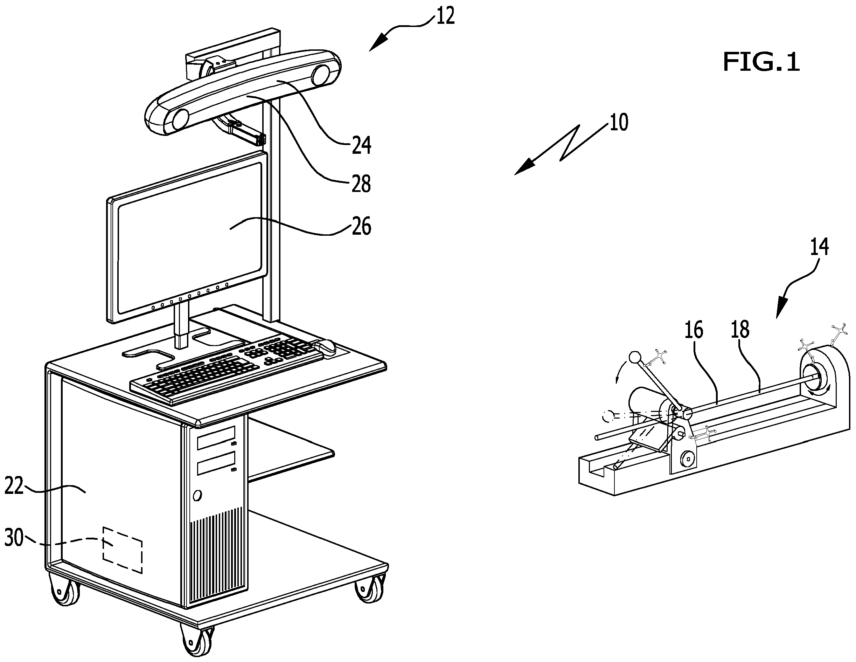

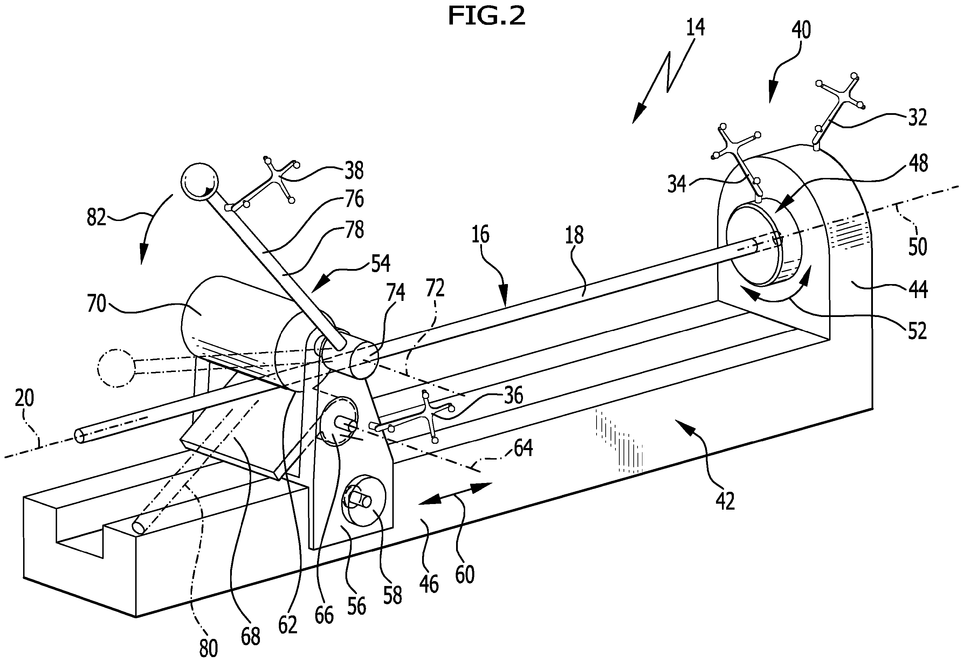

Figur 1 zeigt in perspektivischer schematischer Darstellung ein insgesamt mit dem Bezugszeichen 10 belegtes erfindungsgemäßes Verformungssystem. Das Verformungssystem 10 weist ein medizintechnisches Navigationssystem 12 und eine medizintechnische Verformungseinrichtung 14 auf. Das Verformungssystem 10 dient dazu, einen medizintechnischen Artikel 16 in definierter Weise zu verformen. Vorliegend ist der Artikel 16 als Stabilisierungselement eines medizintechnischen Fixationssystemes ausgestaltet (dies ist selbst in der Zeichnung nicht dargestellt), insbesondere als Stab 18, der eine Achse 20 definiert (Figur 2).

Das Navigationssystem 12 ist in an sich bekannter Weise ausgestaltet und umfasst eine Datenverarbeitungseinrichtung 22, eine Erfassungseinrichtung 24 und eine Hinweiseinrichtung 26. Die Erfassungseinrichtung 24 ist vorliegend ausgestaltet als Navigationskamera 28, beispielsweise als Stereokamera, und mit der Datenverarbeitungseinrichtung 22 gekoppelt. Ebenso ist die Hinweiseinrichtung 26 mit der Datenverarbeitungseinrichtung 22 gekoppelt. Die Datenverarbeitungseinrichtung 22 umfasst eine Speichereinheit 30.

Beispielsweise basierend auf präoperativen oder intraoperativen Planungsdaten ist in der Speichereinheit 30 eine Sollform des Stabes 18 gespeichert. Die Sollform umfasst beispielsweise Informationen darüber, an welchen Verformungspositionen der Stab 18 in welchem Ausmaß zu verformen ist oder verformt sein sollte, damit in der Zeichnung nicht dargestellte Verankerungselemente des Fixationssystems mit dem Stab 18 verbunden werden können.

Zu diesem Zweck kann die Sollform des Stabes 18 in unterschiedliche Segmente unterteilt sein. Zu einem jeweiligen Segment kann gespeichert sein, an welcher Soll-Verformungsposition mittels der Verformungseinrichtung 14 welche Soll-Verformung durchzuführen ist, um die gewünschte Verformung des Stabes 18 sicherzustellen.

Mit dem Navigationssystem 12 können in an sich bekannter Weise medizintechnische Markiereinrichtungen hinsichtlich Lage und/oder Orientierung relativ zum Navigationssystem 12 erfasst und verfolgt werden (Tracking).

Vorliegend sind vier von der Verformungseinrichtung 14 umfasste Markiereinrichtungen 32, 34, 36 und 38 vorgesehen.

Im vorliegenden Fall sind die Markiereinrichtungen 32 bis 38 so unterschiedlich zueinander ausgestaltet, dass sie vom Navigationssystem 12 zweifelsfrei identifiziert und voneinander unterschieden werden können. Das Navigationssystem 12 weist eine Information darüber auf, an welchem Bauteil der Verformungseinrichtung 14 eine jeweilige Markiereinrichtung 32 bis 38 festgelegt ist, worauf nachfolgend noch eingegangen wird.

Die Markiereinrichtung 32 bis 38 sind vorliegend passiv als sogenannte "Rigid Bodys" ausgestaltet, die von der Navigationskamera 28 ausgesandtes Licht reflektieren. Alternativ oder ergänzend ist der Einsatz aktiver Markiereinrichtungen denkbar.

Wie insbesondere aus Figur 2 deutlich wird, ist die Verformungseinrichtung 14 eine Biegeeinrichtung 40 zum Biegen des Stabes 18. Die Biegeeinrichtung 40 umfasst eine sockelartig ausgestaltete Halteeinrichtung 42, die beispielsweise an einer Endseite einen Haltevorsprung 44 aufweist. Der Haltevorsprung 44 springt von einer Führung 46 vor, beispielsweise als Schiene ausgestaltet.

Am Haltevorsprung 44 ist die Markiereinrichtung 32 festgelegt. Auf diese Weise kann die Lage und/oder Orientierung der Biegeeinrichtung 40 insgesamt relativ zum Navigationssystem 12 bestimmt werden. Dies bietet den Vorteil, dass die Biegeeinrichtung 40 während des Einsatzes nicht ortsfest fixiert werden muss, sondern relativ zum Navigationssystem 12 bewegt werden kann.

Am Haltevorsprung 44 ist eine Aufnahme 48 für den Stab 18 festgelegt. Die Aufnahme 48 definiert eine Achse 50, die mit der Achse 20 des in die Aufnahme 48 eingesetzten Stabes 18 fluchtet.

Zum Fixieren des Stabes 18 kann die Aufnahme 48 ähnlich einem Bohrfutter ausgestaltet sein. Der Stab 18 kann in der Aufnahme 48 kraft- und/oder formschlüssig gehalten sein.

Die Aufnahme 48 ist relativ zum Haltevorsprung 44 drehbar ausgestaltet und insbesondere an diesem um die Achse 50 drehbar gelagert (Doppelpfeil 52).

An der Aufnahme 48 ist die Markiereinrichtung 34 gehalten. Eine Rotation des Stabes 18 um die Achse 50 und damit um die Achse 20 des Stabes 18 kann auf diese Weise vom Navigationssystem 12 bestimmt werden.

Die Biegeeinrichtung 40 umfasst ferner ein Verformungswerkzeug 54, das vorliegend insbesondere als Biegewerkzeug zum Biegen des Stabes 18 ausgestaltet ist.

Das Verformungswerkzeug 54 umfasst einen Lagerkörper 56. Der Lagerkörper 56 ist an der Führung 46 verschieblich gelagert und kann längs der Achse 20 verschoben werden. Der Lagerkörper 56 kann beispielsweise mittels eines Feststellelementes 58 unbeweglich an der Führung 46 fixiert werden. Der Doppelpfeil 60 kennzeichnet die Verschieberichtung des Lagerkörpers 56.

Der Lagerkörper 56 ist als Lagerblock ausgestaltet. Ein Anlageelement 62 des Verformungswerkzeugs 54 ist am Lagerkörper 56 gehalten. Das Anlageelement 62 bildet insbesondere ein Auflageelement für den Stab 18. Vorzugsweise ist das Anlageelement 62 so angeordnet, dass der darauf aufliegende Stab 18, wenn in der Aufnahme 48 fixiert, parallel zur Verschieberichtung des Lagerkörpers 56 ausgerichtet ist.

Das Anlageelement 62 ist am Lagerkörper 56 feststellbar beweglich gehalten und insbesondere um eine Schwenkachse 64 schwenkbar gelagert. Zum Feststellen des Anlageelementes 62 ist ein Feststellelement 66 vorgesehen. Die Schwenkachse 64 ist quer zur Achse 50 und zur Führung 46 ausgerichtet.

Das Verformungswerkzeug 54 umfasst ein Anschlagelement 68, um die Verformung des Stabes 18 zu begrenzen. Das Anschlagelement 68 ist vorliegend plattenförmig ausgestaltet und vorzugsweise integral mit dem Anlageelement 62 gebildet, so dass diese gemeinsam einen im Wesentlichen P-förmigen Querschnitt aufweisen. Durch Verschwenken des Anlageelementes 62 um die Schwenkachse 64 kann das Anschlagelement 68 ebenfalls verschwenkt werden, so dass dessen freies Ende näher oder weniger nah an der Führung 46 angeordnet ist. Auf diese Weise wird der Winkel zwischen dem Anlageelement 62 und der Führung 46 und damit auch der Winkel relativ zur Achse 50 verändert.

Es versteht sich, dass die Möglichkeit, das Anschlagelement 68 relativ zum Lagerkörper 56 zu verschwenken, von Vorteil ist. Durch die vorliegend einstückige Ausgestaltung des Anlageelementes 62 mit dem Anschlagelement 68 wird dabei auch das Anlageelement 62 verschwenkt. Sollten die Elemente getrennte Bauteile sein, kann eine unbewegliche Fixierung des Anlageelementes 62 vorgesehen und eine schwenkbare Lagerung des Anschlagelementes 68 am Lagerkörper 56 vorgesehen sein.

Das Verformungswerkzeug 54 umfasst ferner einen Biegekörper 70. Der Biegekörper 70 ist im Abstand zum Anlageelement 62 angeordnet, so dass zwischen diesen ein Zwischenraum zum Hindurchführen des Stabes 18 vorhanden ist. Der Biegekörper 70 ist um eine Schwenkachse 72 am Lagerkörper 56 schwenkbar gelagert. Die Schwenkachse 72 ist quer zur Achse 50 und zur Führung 46 sowie parallel zur Schwenkachse 64 ausgerichtet.

Der Biegekörper 70 ist exzentrisch bezüglich der Schwenkachse 72 angeordnet und beispielsweise von im Wesentlichen zylindrischer Gestalt. Die Schwenkachse 72 wird definiert durch eine Welle 74, an der ein Betätigungselement 76 des Verformungswerkzeugs 54 festgelegt ist. Vorliegend bildet das Betätigungselement 76 einen Hebel 78.

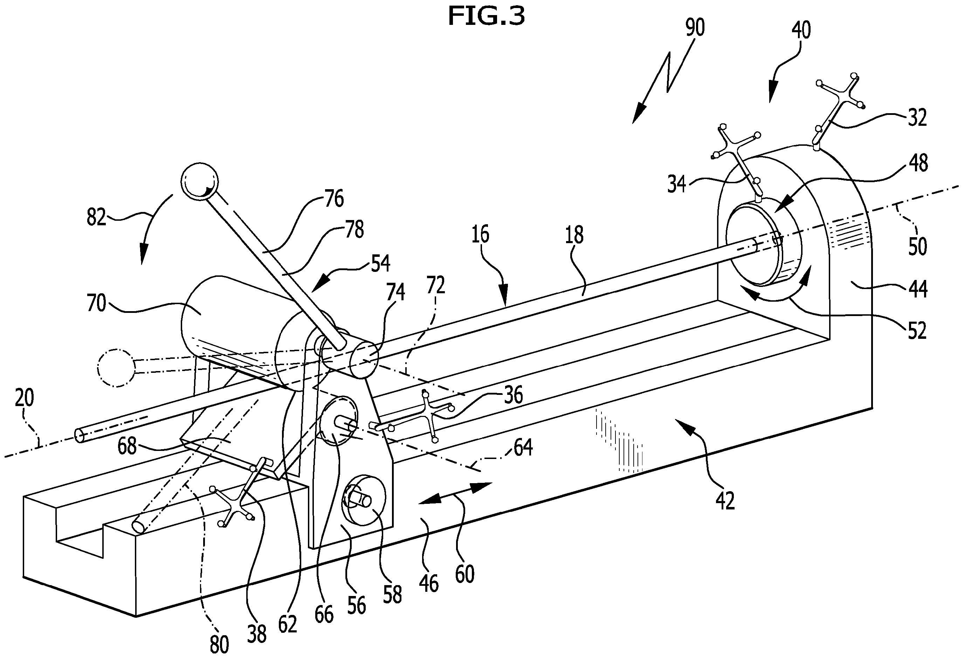

Durch Verschwenken des Betätigungselementes 76 um die Schwenkachse 72 wird der Biegekörper 70 exzentrisch zur Schwenkachse 72 verschwenkt. Dabei kann der Stab 18 mit einer Biegekraft beaufschlagt werden. Der Stab 18 kann solange verbogen werden, bis er am Anschlagelement 68 anschlägt. Dies wird durch die strichpunktierte Kontur 80 in den Figuren 2 und 3 symbolisiert.

Die Markiereinrichtung 36 ist am Lagerkörper 56 festgelegt. Dies erlaubt es dem Navigationssystem 12 festzustellen, in welcher definierten Relativposition der Lagerkörper 56 und damit das Verformungswerkzeug 54 und die Aufnahme 48 angeordnet sind. Beim Verschieben des Lagerkörpers 56 kann diese Änderung der Relativposition ermittelt werden. Da der Stab 18 in der Aufnahme 48 gehalten ist, kann das Navigationssystem 12 auch die Position des Verformungswerkzeuges 54 relativ zum Stab 18 bestimmen.

Bei der Verformungseinrichtung 14 ist die Markiereinrichtung 38 am Betätigungselement 76 festgelegt. Beim Verschwenken des Betätigungselementes 76 kann die Bewegung vom Navigationssystem 12 festgestellt werden. Daraus kann ermittelt werden, in welchem Ausmaß der Biegekörper 70 bewegt wird. Das Navigationssystem 12 kann auf diese Weise berechnen, in welchem Ausmaß der Stab 18 verformt und insbesondere gebogen wird.

Nachfolgend werden die Funktionsweise des Verformungssystems 10 und der Verformungseinrichtung 14 sowie der Verfahrensablauf erläutert.

Der ursprünglich beispielsweise geradlinig erstreckte Stab 18 soll in die in der Speichereinheit 30 gespeicherte Sollform gebracht werden. Hierzu kann, wie erwähnt, die Sollform in mehrere Segmente unterteilt werden und der Stab 18 sukzessive gebogen werden. Dabei wird der Stab 18 vorzugsweise von distal nach proximal gebogen, bezogen auf die Aufnahme 48.

Das Navigationssystem 12 überprüft, beispielsweise unter Verwendung der Markiereinrichtungen 34 und 36, ob der Lagerkörper 56 und damit das Verformungswerkzeug 54 in korrekter Soll-Verformungsposition relativ zur Aufnahme 48 positioniert ist. An der Hinweiseinrichtung 26 kann dem Benutzer ein Hinweis bereitgestellt werden, den Lagerkörper 56 zu verschieben. Nimmt der Lagerkörper 56 die Soll-Verformungsposition ein, kann ein entsprechender Hinweis an der Hinweiseinrichtung 56 bereitgestellt werden.

Vorliegend kommt als Hinweiseinrichtung 26 der Bildschirm des Navigationssystems 12 zum Einsatz. Außer einer optischen Ausgabe eines Hinweises ist eine akustische Ausgabe eines Hinweises denkbar.

Anschließend wird der Stab 18 an der Soll-Verformungsposition gebogen, indem das Betätigungselement 76 mit einer Biegekraft beaufschlagt wird (Pfeil 82). Die Bewegung des Betätigungselementes 76 wird vom Navigationssystem 12 verfolgt. Ist der Soll-Biegewinkel erreicht, ermittelt anhand der Bewegung des Betätigungselementes 76, kann an der Hinweiseinrichtung 26 ein entsprechender Hinweis bereitgestellt werden. Das Navigationssystem 12 kann auf diese Weise feststellen, dass das erste Segment die korrekte Sollform einnimmt.

Im weiteren Verlauf des Verfahrens kann der Lagerkörper 56 längs der Führung 46 weiter verschoben werden, bis zu einer nachfolgenden Soll-Verformungsposition. Nach deren Erreichen, begleitet von einem entsprechenden Hinweis an den Benutzer, kann abermals eine Biegung durchgeführt werden. Bei Erreichen des richtigen Biegewinkels wird ein Hinweis bereitgestellt. Auf diese Weise wird die Sollform des zweiten Segmentes überprüft.

Sukzessive kann der Stab 18 nun durch Verschieben des Lagerkörpers 56 und Biegen des Stabes 18 in die gewünschte Sollform überführt werden. Vorteilhafterweise wird vor jeder Biegung mittels des Feststellelementes 58 sichergestellt, dass die Soll-Verformungsposition des Lagerkörpers 56 erhalten bleibt.

Im vorstehend genannten Beispiel wird der Stab 18 in einer Ebene gebogen. Ist die Biegung in voneinander unterschiedlichen Ebenen durchzuführen, kann vor einer jeweiligen Biegung die Biegeebene durch Rotation des Stabes 18 um die Achse 20 geändert werden. Die Rotation kann vom Navigationssystem 12 anhand der Markiereinrichtung 34 bestimmt werden. Ist die korrekte Biegeebene erreicht, kann auch in diesem Fall an der Hinweiseinrichtung 26 ein korrespondierender Hinweis ausgegeben werden.

Auf vorstehend genannte Weise besteht bei der erfindungsgemäßen Verformungseinrichtung 14 die Möglichkeit, jeden Freiheitsgrad mittels des Navigationssystems 12 zu überwachen, nämlich Drehung des Stabes 18 um die Achse 50, Biegeposition durch Verschiebung des Verformungswerkzeuges 54 und Biegewinkel mittels Verschwenkung des Betätigungselementes 76. Zusätzlich kann die Position der Verformungseinrichtung 14 im Raum relativ zum Navigationssystem 12 bestimmt werden.

Figur 3 zeigt in einer der Figur 2 entsprechenden Weise eine mit dem Bezugszeichen 90 belegte vorteilhafte Ausführungsform einer erfindungsgemäßen Verformungseinrichtung, die anstelle der oder zusammen mit der Verformungseinrichtung 14 Bestandteil eines erfindungsgemäßen Navigationssystems sein könnte.

Für gleiche oder gleichwirkende Merkmale und Bauteile werden identische Bezugszeichen benutzt. Die mit der Verformungseinrichtung 14 erzielbaren Vorteile können bei der Verformungseinrichtung 90 ebenfalls erzielt werden, so dass auf die voranstehenden Ausführungen verwiesen werden kann. Es werden nur die wesentlichen Unterschiede erläutert.

Bei der Verformungseinrichtung 90 entfällt die Markiereinrichtung am Betätigungselement 76. Stattdessen ist die Markiereinrichtung 38 am Anschlagelement 68 gehalten. Auf diese Weise kann das Navigationssystem 12 feststellen, in welchem Umfang der Stab 18 maximal gebogen werden kann, unter der Annahme, dass der Stab 18 tatsächlich bis zum Anschlag am Anschlagelement 68 verbogen wird.

Vor der Biegung kann der Benutzer das Anschlagelement 68 um die Schwenkachse 64 verschwenken, bevorzugt anhand eines Hinweises des Navigationssystems 12. Nimmt das Anschlagelement 68 die korrekte Solllage für die zu erfolgende Biegung ein, kann ein diesbezüglicher Hinweis bereitgestellt werden. Der Benutzer kann das Anschlagelement 68 mittels des Feststellelementes 66 fixieren. Anschließend kann der Stab 18 gebogen werden. Bei Anschlag am Anschlagelement 68 weiß der Benutzer, dass der gewünschte Biegewinkel erreicht ist.

Es kann selbstverständlich vorgesehen sein, dass sowohl am Betätigungselement 76 als auch am Anschlagelement 68 eine jeweilige Markiereinrichtung gehalten ist.

### Bezugszeichenliste:

- 10: Verformungssystem
- 12: Navigationssystem
- 14: Verformungseinrichtung
- 16: Artikel
- 18: Stab
- 20: Achse
- 22: Datenverarbeitungseinrichtung
- 24: Erfassungseinrichtung
- 26: Hinweiseinrichtung
- 28: Navigationskamera
- 30: Speicheinheit
- 32: Markiereinrichtung
- 34: Markiereinrichtung
- 36: Markiereinrichtung
- 38: Markiereinrichtung
- 40: Biegeeinrichtung
- 42: Halteeinrichtung
- 44: Haltevorsprung
- 46: Führung
- 48: Aufnahme
- 50: Achse
- 52: Doppelpfeil
- 54: Verformungswerkzeug
- 56: Lagerkörper
- 58: Feststellelement
- 60: Doppelpfeil
- 62: Anlageelement
- 64: Schwenkachse
- 66: Feststellelement
- 68: Anschlagelement
- 70: Biegekörper
- 72: Schwenkachse
- 74: Welle
- 76: Betätigungselement
- 78: Hebel
- 80: Kontur
- 82: Pfeil
- 90: Verformungseinrichtung

## Patentansprüche

1. Medizintechnische Verformungseinrichtung für einen medizintechnischen Artikel, die eine Aufnahme (48) zum Fixieren des Artikels (16) aufweist und ein Verformungswerkzeug (54), mit dem zum Verformen auf den Artikel (16) eingewirkt wird, wobei das Verformungswerkzeug (54) in definierter Weise relativ zur Aufnahme (48) bewegbar ist und an der Aufnahme (48) und am Verformungswerkzeug (54) jeweils eine von einem Navigationssystem (12) erfassbare medizintechnische Markiereinrichtung (32, 34, 36, 38) gehalten ist zum Bestimmen einer Relativposition des Verformungswerkzeugs (54) und der Aufnahme (48), und wobei am Verformungswerkzeug (54) eine weitere Markiereinrichtung (32, 34, 36, 38) festgelegt ist zum Bestimmen, mittels des Navigationssystems (12), eines Ausmaßes einer Verformung des Artikels (16).

2. Verformungseinrichtung nach Anspruch 1, **dadurch gekennzeichnet, dass** das Verformungswerkzeug (54) relativ zur Aufnahme (48) verschieblich ausgestaltet ist.

3. Verformungseinrichtung nach Anspruch 1 oder 2, **dadurch gekennzeichnet, dass** die Verformungseinrichtung (14; 90) eine Halteeinrichtung (42) umfasst, die die Aufnahme (48) umfasst oder ausbildet oder an der die Aufnahme (48) festgelegt ist, und dass das Verformungswerkzeug (54) an der Halteeinrichtung (42) vorzugsweise feststellbar beweglich gehalten ist.

4. Verformungseinrichtung nach Anspruch 3, **dadurch gekennzeichnet, dass** die Halteeinrichtung (42) eine Führung (46) für einen Lagerkörper (56) des Verformungswerkzeuges (54) umfasst oder bildet, wobei vorzugsweise eine Markiereinrichtung (32, 34, 36, 38) der Verformungseinrichtung (10; 90) am Lagerkörper (56) gehalten ist.

5. Verformungseinrichtung nach Anspruch 3 oder 4, **dadurch gekennzeichnet, dass** die Verformungseinrichtung (10; 90) eine an der Halteeinrichtung (42) gehaltene medizintechnische Markiereinrichtung (32, 34, 36, 38) umfasst zum Bestimmen der Lage und/oder Orientierung der Verformungseinrichtung (10; 90) relativ zum Navigationssystem (12).

6. Verformungseinrichtung nach einem der Ansprüche 3 bis 5, **dadurch gekennzeichnet, dass** die Aufnahme (48) relativ zur Halteeinrichtung (42) drehbar ausgestaltet ist, insbesondere an der Halteeinrichtung (42) drehbar gelagert ist.

7. Verformungseinrichtung nach Anspruch 6, **dadurch gekennzeichnet, dass** die Aufnahme (48) eine Achse (50) definiert, die mit einer vom in der Aufnahme (48) fixierten längserstreckten Artikel (16) definierten Achse (20) fluchtet, und/oder **gekennzeichnet durch** eine handbetätigte oder mittels eines Antriebs betätigte Drehung der Aufnahme (48) relativ zur Halteeinrichtung (42).

8. Verformungseinrichtung nach einem der voranstehenden Ansprüche, **gekennzeichnet durch** eine handbetätigte oder mittels eines Antriebs betätigte Bewegung und insbesondere Verschiebung des Verformungswerkzeugs (54) relativ zur Aufnahme (48) und/oder **gekennzeichnet durch** ein von Hand oder mittels eines Antriebs betätigbares Verformungswerkzeug (54).

9. Verformungseinrichtung nach einem der voranstehenden Ansprüche, **dadurch gekennzeichnet, dass** das Verformungswerkzeug (54) ein Biegewerkzeug ist und einen Biegekörper (70) und ein mit diesem gekoppeltes Betätigungselement (76) umfasst, wobei der Artikel (16) mittels des Biegekörpers (70) gebogen werden kann, wobei das Betätigungselement (76) vorzugsweise an einem Lagerkörper (56) des Biegewerkzeugs drehbar und/oder verschieblich gelagert ist.

10. Verformungseinrichtung nach einem der voranstehenden Ansprüche, **dadurch gekennzeichnet, dass** das Verformungswerkzeug (54) ein Anlageelement (62) und insbesondere ein Auflageelement für den Artikel (16) umfasst oder ausbildet und/oder dass das Verformungswerkzeug (54) ein verstellbares Anschlagelement (68) für den Artikel (16) umfasst oder ausbildet, zum Begrenzen der Verformung des Artikels (16), vorzugsweise dass das Anschlagelement (68) an einem Lagerkörper (56) des Verformungswerkzeuges (54) feststellbar beweglich gehalten ist.

11. Verformungseinrichtung nach Anspruch 9 oder 10, **dadurch gekennzeichnet, dass** am Betätigungselement (76) und/oder am Anschlagelement (68) eine Markiereinrichtung (32, 34, 36, 38) des Verformungswerkzeuges (54) gehalten ist.

12. Medizintechnisches Verformungssystem, umfassend eine Verformungseinrichtung (14; 90) nach einem der voranstehenden Ansprüche und ein medizintechnisches Navigationssystem (12) zum Erfassen und Verfolgen der Markiereinrichtungen (32, 34, 36, 38) der Verformungseinrichtung (14; 90), wobei mit dem Navigationssystem (12) die Relativposition der Aufnahme (48) und des Verformungswerkzeugs (54) ermittelbar ist zum Feststellen einer Verformungsposition am Artikel (16), und wobei mit dem Navigationssystem (12) das Ausmaß der Verformung des Artikels (16) an der Verformungsposition ermittelbar ist.

13. Verformungssystem nach Anspruch 12, **dadurch gekennzeichnet, dass** in einer Speichereinheit (30) eine Sollform des Artikels (16) gespeichert ist und dass das Navigationssystem (12) eine Hinweiseinrichtung (26) umfasst oder ausbildet zum Bereitstellen mindestens eines Hinweises darüber:
- dass das Verformungswerkzeug (54) eine Soll-Verformungsposition zur Aufnahme (48) einnimmt, insbesondere beim Bewegen des Verformungswerkzeuges (54) relativ zur Aufnahme (48);
- dass die mittels des Verformungswerkzeuges erzeugte oder erzeugbare Verformung des Artikels (16) mit einer Soll-Verformung übereinstimmt.

14. Verformungssystem nach Anspruch 13, **dadurch gekennzeichnet, dass** die Sollform vom Navigationssystem (12) in einzelne Segmente unterteilbar ist, wobei an der Hinweiseinrichtung (26) sequentiell Hinweise bereitstellbar sind, dass ein jeweiliges Segment durch Verformen des Artikels an einer Soll-Verformungsposition mit einer Soll-Verformung mit einer Sollform des jeweiligen Segmentes übereinstimmt und/oder dass vom Navigationssystem (12) Verformungsinformationen an mindestens einen Antrieb der Verformungseinrichtung (14; 90) zum maschinellen Verformen des Artikels (16) entsprechend der Verformungsinformationen übermittelbar sind.

15. Verfahren zum Verformen eines medizintechnischen Artikels mit einem Verformungssystem nach einem der Ansprüche 12 bis 14, **dadurch gekennzeichnet, dass** mit dem Navigationssystem eine Verformungsposition des zu verformenden medizintechnischen Artikels festgestellt wird und ein Ausmaß der Verformung des Artikels an der Verformungsposition ermittelt wird.

## Claims

1. Medical deformation device for a medical article, said deformation device comprising a receptacle (48) for fixing the article (16), a deformation tool (54) used to act on the article (16) for deformation purposes, the deformation tool (54) being movable in a defined manner relative to the receptacle (48), and a medical marking device (32, 34, 36, 38) that is detectable by a navigation system (12) is held in each case on the receptacle (48) and on the deformation tool (54) for the purposes of determining a relative position of the deformation tool (54) and the receptacle (48), and wherein a further marking device (32, 34, 36, 38) is fixed to the deformation tool (54) for the purposes of determining, by means of the navigation system (12), an extent of a deformation of the article (16).

2. Deformation device in accordance with claim 1, **characterized in that** the deformation tool (54) is configured to be displaceable relative to the receptacle (48).

3. Deformation device in accordance with claim 1 or 2, **characterized in that** the deformation device (14; 90) comprises a holding device (42), which comprises or forms the receptacle (48) or on which the receptacle (48) is fixed, and **in that** the deformation tool (54) is held in movable manner on the holding device (42), preferably in a securable manner.

4. Deformation device in accordance with claim 3, **characterized in that** the holding device (42) comprises or forms a guide (46) for a bearing body (56) of the deformation tool (54), wherein, preferably, a marking device (32, 34, 36, 38) of the deformation device (10; 90) is held on the bearing body (56).

5. Deformation device in accordance with claim 3 or 4, **characterized in that** the deformation device (10; 90) comprises a medical marking device (32, 34, 36, 38) held on the holding device (42) for the purposes of determining the position and/or orientation of the deformation device (10; 90) relative to the navigation system (12).

6. Deformation device in accordance with claims 3 to 5, **characterized in that** the receptacle (48) is configured to be rotatable relative to the holding device (42), in particular being rotatably mounted on the holding device (42).

7. Deformation device in accordance with claim 6, **characterized in that** the receptacle (48) defines an axis (50) that is aligned with an axis defined by the article (16), said article having a longitudinal extension and being fixed in the receptacle (48), and/or **characterized by** a rotation of the receptacle (48) relative to the holding device (42), said rotation being actuated manually or by means of a drive.

8. Deformation device in accordance with one of the preceding claims, **characterized by** a movement and, in particular, a displacement of the deformation tool (54) relative to the receptacle (48) which is actuated manually or by means of a drive and/or **characterized by** a deformation tool (54) that is actuatable by hand or by means of a drive.

9. Deformation device in accordance with one of the preceding claims, **characterized in that** the deformation tool (54) is a bending tool and comprises a bending body (70) and, coupled therewith, an actuation element (76), wherein the article (16) can be bent by means of the bending body (70), wherein the actuation element (76) is preferably mounted in rotatable and/or displaceable manner on a bearing body (56) of the bending tool.

10. Deformation device in accordance with one of the preceding claims, **characterized in that** the deformation tool (54) comprises or forms an abutment element (62) and, in particular, a support element for the article (16) and/or that the deformation tool (54) comprises or forms an adjustable stop element (68) for the article (16) for the purposes of restricting the deformation of the article (16), preferably **in that** the stop element (68) is held in a movable, securable manner on a bearing body (56) of the deformation tool (54).

11. Deformation device in accordance with claim 9 or 10, **characterized in that** a marking device (32, 34, 36, 38) of the deformation tool (54) is held on the actuation element (76) and/or on the stop element (68).

12. Medical deformation system, comprising a deformation device (14, 90) in accordance with one of the preceding claims and a medical navigation system (12) for detecting and tracking the marking devices (32, 34, 36, 38) of the deformation device (14; 90), wherein the relative position of the receptacle (48) and of the deformation tool (54) is determinable by the navigation system (12) for the purposes of determining a deformation position on the article (16), and wherein the extent of the deformation of the article (16) at the deformation position is determinable with the navigation system (12).

13. Deformation system in accordance with claim 12, **characterized in that** a desired shape of the article (16) is stored in a memory unit (30) and **in that** the navigation system (12) comprises or forms a notification device (26) for providing at least one notification in respect of:
- the deformation tool (54) assuming a desired deformation position relative to the receptacle (48), particularly when moving the deformation tool (54) relative to the receptacle (48);
- the deformation of the article (16) that is produced or producible by means of the deformation tool coinciding with a desired deformation.

14. Deformation system in accordance with claim 13, **characterized in that** the desired shape is subdividable into individual segments by the navigation system (12), wherein notifications are providable in sequence at the notification device (26) that, by deformation of the article at a desired deformation position with a desired deformation, a respective segment coincides with a desired shape of the respective segment, and/or that deformation information items are transmittable from the navigation system (12) to at least one drive of the deformation device (14; 90) for the purposes of a machine deformation of the article (16) in accordance with the deformation information items.

15. Method for deforming a medical article using a navigation system in accordance with one of claims 12 to 14, **characterized in that** the navigation system is used to determine a deformation position of the medical article to be deformed and an extent of the deformation of the article at the deformation position is ascertained.

## Revendications

1. Dispositif de déformation médico-technique pour un article médico-technique, qui présente un logement (48) pour fixer l'article (16) et un outil de déformation (54) avec lequel on agit sur l'article (16) pour le déformer, dans lequel l'outil de déformation (54) est mobile d'une manière définie par rapport au logement (48) et un dispositif de marquage (32, 34, 36, 38) médico-technique détectable par un système de navigation (12) est maintenu respectivement contre le logement (48) et l'outil de déformation (54) pour établir une position relative de l'outil de déformation (54) et du logement (48), et dans lequel un autre dispositif de marquage (32, 34, 36, 38) est fixé contre l'outil de déformation (54) pour établir, au moyen du système de navigation (12), un degré de déformation de l'article (16).

2. Dispositif de déformation selon la revendication 1, **caractérisé en ce que** l'outil de déformation (54) est conçu de façon coulissante par rapport au logement (48).

3. Dispositif de déformation selon la revendication 1 ou 2, **caractérisé en ce que** le dispositif de déformation (14 ; 90) comprend un dispositif de maintien (42) qui comprend ou forme le logement (48) ou contre lequel est fixé le logement (48), et **en ce que** l'outil de déformation (54) est maintenu en mouvement et de préférence fixable contre le dispositif de maintien (42).

4. Dispositif de déformation selon la revendication 3, **caractérisé en ce que** le dispositif de maintien (42) comprend ou forme un guidage (46) pour un corps de palier (56) de l'outil de déformation (54), dans lequel de préférence un dispositif de marquage (32, 34, 36, 38) du dispositif de déformation (10 ; 90) est maintenu contre le corps de palier (56).

5. Dispositif de déformation selon la revendication 3 ou 4, **caractérisé en ce que** le dispositif de déformation (10 ; 90) comprend un dispositif de marquage médico-technique (32, 34, 36, 38) maintenu contre le dispositif de maintien (42) pour établir la position et/ou l'orientation du dispositif de déformation (10 ; 90) par rapport au système de navigation (12).

6. Dispositif de déformation selon l'une des revendications 3 à 5, **caractérisé en ce que** le logement (48) est conçu en rotation par rapport au dispositif de maintien (42), en particulier est monté en rotation contre le dispositif de maintien (42).

7. Dispositif de déformation selon la revendication 6, **caractérisé en ce que** le logement (48) définit un axe (50) qui est aligné sur un axe défini par l'article (16) s'étendant longitudinalement et fixé dans le logement (48), et/ou **caractérisé par** une rotation du logement (48), actionnée manuellement ou actionnée au moyen d'un entraînement, par rapport au dispositif de maintien (42).

8. Dispositif de déformation selon l'une des revendications précédentes, **caractérisé par** un mouvement et en particulier un déplacement, actionné manuellement ou actionné au moyen d'un entraînement, de l'outil de déformation (54) par rapport au logement (48) et/ou **caractérisé par** un outil de déformation (54) pouvant être actionné manuellement ou au moyen d'un entraînement.

9. Dispositif de déformation selon l'une des revendications précédentes, **caractérisé en ce que** l'outil de déformation (54) est un outil de pliage et comprend un corps de pliage (70) et un élément d'actionnement (76) couplé à celui-ci, dans lequel l'article (16) peut être plié au moyen du corps de pliage (70), dans lequel l'élément d'actionnement (76) est monté en rotation et/ou en déplacement de préférence contre un corps de palier (56) de l'outil de pliage.

10. Dispositif de déformation selon l'une des revendications précédentes, **caractérisé en ce que** l'outil de déformation (54) comprend ou forme un élément d'application (62) et en particulier un élément d'appui pour l'article (16) et/ou **en ce que** l'outil de déformation (54) comprend ou forme un élément de butée (68) réglable pour l'article (16), pour délimiter la déformation de l'article (16), de préférence **en ce que** l'élément de butée (68) est maintenu en mouvement et fixable contre un corps de palier (56) de l'outil de déformation (54).

11. Dispositif de déformation selon la revendication 9 ou 10, **caractérisé en ce qu'**un dispositif de marquage (32, 34, 36, 38) de l'outil de déformation (54) est maintenu contre l'élément d'actionnement (76) et/ou contre l'élément de butée (68).

12. Système de déformation médico-technique, comprenant un dispositif de déformation (14 ; 90) selon l'une des revendications précédentes et un système de navigation médico-technique (12) pour détecter et suivre les dispositifs de marquage (32, 34, 36, 38) du dispositif de déformation (14 ; 90), dans lequel la position relative du logement (48) et de l'outil de déformation (54) peut être déterminée avec le système de navigation (12) pour déterminer une position de déformation contre l'article (16), et dans lequel le degré de déformation de l'article (16) au niveau de la position de déformation peut être déterminé avec le système de navigation (12).

13. Système de déformation selon la revendication 12, **caractérisé en ce que** dans une unité de mémorisation (30) est mémorisée une forme desirée de l'article (16) et **en ce que** le système de navigation (12) comprend ou forme un dispositif indicateur (26) pour fournir au moins une indication signalant que :
- l'outil de déformation (54) adopte une position de déformation desirée par rapport au logement (48), en particulier lors du mouvement de l'outil de déformation (54) par rapport au logement (48) ;
- la déformation de l'article (16) produite ou pouvant être produite au moyen de l'outil de déformation correspond à une déformation desirée.

14. Système de déformation selon la revendication 13, **caractérisé en ce que** la forme desirée peut être divisée en segments individuels par le système de navigation (12), dans lequel des indications peuvent être séquentiellement fournies au niveau du dispositif indicateur (26) signalant que, par déformation de l'article au niveau d'une position de déformation desirée avec une déformation desirée, un segment respectif correspond à une forme desirée du segment respectif, et/ou **en ce que** des informations de déformation au niveau d'au moins un entraînement du dispositif de déformation (14 ; 90) pour déformer mécaniquement l'article (16) peuvent être transmises par le système de navigation (12) conformément aux informations de déformation.

15. Procédé de déformation d'un article médico-technique avec un système de déformation selon l'une des revendications 12 à 14, **caractérisé en ce qu'**avec le système de navigation une position de déformation de l'article médico-technique à déformer est déterminée et un degré de déformation de l'article au niveau de la position de déformation est déterminé.
